# EUROPÄISCHE PATENTSCHRIFT

(11) **EP 2 996 594 B1**
(45) Veröffentlichungstag und Bekanntmachung des Hinweises auf die Patenterteilung: **17.11.2021**
(21) Anmeldenummer: 14724411.5
(22) Anmeldetag: 13.05.2014
(51) Int. Cl.: A61B 17/80

(54) **OSTEOSYNTHESEPLATTE SOWIE SYSTEM ZUR OSTEOSYNTHESE**
OSTEOSYNTHESIS PLATE AND SYSTEM FOR OSTEOSYNTHESIS
PLAQUE D'OSTÉOSYNTHÈSE ET SYSTÈME D'OSTÉOSYNTHÈSE

(30) Priorität: 13.05.2013 DE 102013104887
(43) Veröffentlichungstag der Anmeldung: 23.03.2016
(73) Patentinhaber: AAP Implantate AG, 12099 Berlin (DE)
(72) Erfinder: REUTER, Andreas, 14482 Potsdam (DE)
(74) Vertreter: Friderichs, Gunther
(86) Internationale Anmeldenummer: PCT/EP2014/059726
(87) Internationale Veröffentlichungsnummer: WO 2014/184175

(56) Entgegenhaltungen:
- EP-A2- 1 393 689
- WO-A1-99/37231
- WO-A1-2013/029188
- WO-A2-2004/045389
- WO-A2-2012/135860
- DE-A1-102010 041 564
- DE-U1-202008 014 358
- FR-A1- 2 956 972
- GB-A- 2 422 783
- US-A1- 2006 089 648
- US-A1- 2006 100 625
- US-A1- 2006 142 767
- US-A1- 2009 118 768
- None

## Beschreibung

### Gebiet der Erfindung

Die Erfindung betrifft eine Osteosyntheseplatte, ein Segment für eine Osteosyntheseplatte sowie ein System zur Osteosynthese.

Insbesondere betrifft die Erfindung ein System zur Osteosynthese von periprothetischen Frakturen und zur Unterstützung bei Frakturen von osteoporotischen Knochen.

### Hintergrund der Erfindung

Systeme zur Osteosynthese, welche eine Knochenplatte umfassen, sind bekannt.

So zeigt beispielsweise die Offenlegungsschrift DE 10 2010 025001 A1 eine Knochenplatte.

Eine derartige Knochenplatte besteht in der Regel aus einem rigiden Material, insbesondere aus Metall oder einem chirurgischen Edelstahl. Die Knochenplatte weist Durchführungen auf, in welche Schrauben eingebracht werden können.

Zumeist werden zur Befestigung der Knochenplatte an den Knochenfragmenten Schrauben verwendet. Um die Schrauben sicher verankern zu können, sollten diese bikortikal eingesetzt werden.

Hierzu umfasst ein Osteosynthesesystem Schrauben in verschiedenen Längen.

Problematisch ist die Versorgung von periprothetischen Frakturen. In diesem Fall kann zumeist die Schraube nicht bikortikal eingesetzt werden, da der Markraum aufgrund der Prothese blockiert ist.

Oft werden daher monokortikale Schrauben verwendet. Eine derartige Verbindung ist aber mechanisch weit weniger belastbar. In vielen Fällen ist aufgrund des geringen Abstandes der Knochenplatte zur Prothese noch nicht einmal die Verwendung von monokortikalen Schrauben möglich. Es kommen daher Cerclage-Drähte zum Einsatz, welche um den Knochen gewickelt werden. Eine derartige Befestigung ist ebenfalls mechanisch nicht besonders stabil und führt zu Verletzungen des Periosts.

Die Offenlegungsschrift US 2010/0262194 A1 zeigt ein Osteosynthesesystem mit an der Knochenplatte anbringbaren Zusatzbauteilen, über welche Knochenschrauben von der Seite her eingeführt werden können, um diese am Markkanal vorbei zu führen.

Dieses System ist aber relativ umständlich, passt sich in vielen Fällen der jeweiligen Anatomie des Patienten nicht hinreichend an und trägt stark auf.

Das Dokument DE 10 2010 041564 A1 zeigt einen anclipsbaren Röntgenmarker für eine Knochenplatte. Das Dokument GB 2 422 783 A zeigt eine Knochenplatte mit einem an der Knochenplatte anbringbaren Ausleger.

### Aufgabe der Erfindung

Der Erfindung liegt demgegenüber die Aufgabe zugrunde, eine Osteosyntheseplatte sowie ein System zur Osteosynthese zur Verfügung zu stellen, welches auf einfache Weise der jeweiligen Knochenform des Patienten angepasst werden kann, insbesondere um bei der Versorgung periprothetischer Frakturen verwendet zu werden.

### Zusammenfassung der Erfindung

Die Aufgabe der Erfindung wird bereits durch eine Osteosyntheseplatte nach Anspruch 1 gelöst.

Die Erfindung betrifft eine Osteosyntheseplatte, welche ein erstes Segment und ein zweites Segment umfasst und wobei beide Segmente ein Loch zur Durchführung einer Knochenschraube aufweisen.

Gemäß der Erfindung ist das zweite Segment lösbar mit dem ersten Segment verbindbar.

Das erste Segment kann also mit dem zweiten Segment verbunden werden. So ist es möglich, je nach Einsatzort Segmente anzubringen oder zu entfernen, so dass die durch die Segmente gebildete Osteosyntheseplatte von Ihrer Grundform her durch das hinzufügen oder abnehmen von Segmenten verändert werden kann.

Vorzugsweise sind die Segmente werkzeuglos verbindbar, es wird insbesondere vorzugsweise keine Schraube zum Verbinden der Segmente verwendet.

Weiter ist der Winkel, in welchem erstes und zweites Segment zu einander angeordnet sind, veränderbar. Unter dem Winkel wird der Winkel verstanden, in welchem die Oberflächen der beiden Segmente zueinander stehen. Sofern es sich um eine gekrümmte Oberfläche handelt kann eine beliebige Tangentenfläche zu dieser Oberfläche als Referenz verwendet werden.

Der Winkel ist vorzugsweise senkrecht zur Plattenebene, also senkrecht zur Oberfläche beziehungsweise einer Tangentenfläche einer gekrümmten Oberfläche veränderbar.

Vorzugsweise lässt sich der Winkel in einem Bereich von mindestens 10°, vorzugsweise in einem Bereich von mindestens 20° verändern.

So ist es möglich, dass erstes und zweites Segment Teil einer Knochenplatte sind und dass sich die Segmente der Kontur des Knochens anpassen.

Vorzugsweise sind die Segmente entlang einer Achse bewegbar, um den Winkel zwischen den Segmenten zu verändern.

Vorzugsweise ist das zweite Segment also schwenkbar an dem ersten Segment angeordnet. Die Schwenkachse verläuft dabei im Wesentlichen parallel zu einer Oberfläche des ersten und zweiten Segments, so dass das zweite Segment flügelartig gegenüber dem ersten Segment bewegbar ist.

Insbesondere sind erstes und zweites Segment mittels eines Scharniers schwenkbar miteinander verbunden.

Die beiden Segmente der Knochenplatte sind also beweglich in mindestens einem, vorzugsweise in genau einem Freiheitsgrad an einer Kante miteinander verbunden.

Dies ermöglicht, dass die Segmente aus rigidem Material der Form des Knochens folgen können.

Gleichzeitig wird durch eine scharnierartige Verbindung mit nur einem Freiheitsgrad sichergestellt, dass das erste Segment gegenüber dem zweiten Segment allenfalls in einer Richtung beweglich ist.

Diese bewegliche Richtung kann beispielsweise parallel zur Fraktur verlaufen, so dass in der Richtung quer zur Fraktur, in welcher die Knochenplatte den Bruch stabilisieren soll, eine feste Verbindung vorhanden ist.

Es ist insbesondere vorgesehen, dass das erste Segment als längliche Knochenplatte mit einer Mehrzahl von Durchführungen ausgebildet ist.

Weitere Segmente können an dem länglichen, als Knochenplatte ausgebildeten Segment beweglich angebracht werden, so dass diese von der Haupterstreckungsrichtung des ersten Segments seitlich abstehen.

Vorzugsweise weist das erste Segment eine Mehrzahl von Befestigungsstellen auf, an denen weitere Segmente beweglich angebracht werden können. Je nach den anatomischen Gegebenheiten des Patienten kann so die Knochenplatte zur Seite hin quasi verlängert werden, so dass benachbart zur Knochenplatte Befestigungsschrauben eingebracht werden können, etwa um bei der Befestigung den Markkanal auszusparen oder die Schrauben in Stellen des Knochens eindrehen zu können, die eine stabilere Knochenstruktur aufweisen.

Bei einer Ausführungsform der Erfindung sind erstes und zweites Segment werkzeuglos trenn- und/oder verbindbar ausgebildet.

Es ist insbesondere vorgesehen, dass das erste Segment, welches als Knochenplatte ausgebildet ist und eine Mehrzahl von Durchführungen aufweist, eine Mehrzahl von Stegen zur Aufnahme einer Scharnieröffnung aufweist.

Unter einem Steg wird ein Abschnitt der Knochenplatte verstanden, welcher als Lager für ein Scharnier dienen kann.

Es ist weiter vorgesehen, dass das zweite Segment einen klammerförmig ausgebildeten Scharnieröffnung aufweist, mit dem dieses an dem ersten Segment angebracht werden kann.

Es ist weiter vorgesehen, dass Segment klammerartig auszubilden, so dass dieses auf einen Steg aufgeschoben werden kann und dort einrastet oder zumindest klemmt.

Dies hat den Vorteil, dass nunmehr die beiden Segmente auch im nicht montierten Zustand in jedem Winkel aneinander befestigt sind. Auf der anderen Seite kann eine klammerartige Verbindung durch den Verwender auch leicht wieder gelöst werden.

Um eine derartige klammerartige Verbindung ausbilden zu können, ist das zweite Segment U-förmig ausgebildet, wobei die zwei Hälften der U-förmigen Ausgestaltung wie eine Feder wirken, so dass ein klammerartig ausgebildeter Scharnierflügel es zulässt, dass das Segment aufgeschoben werden kann, indem sich die U-förmige Ausgestaltung aufweitet und sodann einrastet.

Bei einer Weiterbildung der Erfindung sind eine Vielzahl von Segmenten, also mindestens drei Segmente, aneinander anreihbar.

Hierzu ist insbesondere vorgesehen, dass das zweite Segment sowohl eine Scharnieröffnung als auch einen Steg zur Anbringung eines weiteren Scharniersegmentes aufweist. Auf diese Weise kann der Befestigungsbereich zur Seite hin durch das Aneinanderreihen von Segmenten verlängert werden, wobei diese panzerkettenartig der Kontur des Knochens folgen. Die durch die Segmente gebildete Knochenplatte ist so als Gliederkette ausgebildet.

Es ist denkbar, dass mittels einer so ausgebildeten gliederartigen Kette aus Segmenten der Knochen ganz oder teilweise umschlungen wird.

Die Durchführungen des ersten und/oder des zweiten Segmentes sind vorzugsweise zumindest teilweise als Gewinde ausgebildet, um mit einer entsprechenden Schraube mit einem Gewinde am Kopf eine winkelstabile Verbindung auszubilden.

Bei einer Weiterbildung der Erfindung sind erstes und zweites Segment gegeneinander festlegbar ausgebildet. Hierunter wird verstanden, dass die scharnierartige Verbindung der Segmente im eingesetzten Zustand blockiert werden kann, so dass diese nicht, beziehungsweise nur mit hohem Kraftaufwand, gegeneinander bewegbar sind.

Dies kann insbesondere dadurch erfolgen, dass ein Segment U-förmig ausgebildet ist, wobei beim Einschrauben der Knochenschraube die durch das U gebildeten Hälften gegeneinander gepresst werden, so dass eine kraftschlüssige Verbindung am Steg entsteht.

Das Segment die eine Osteosyntheseplatte kann wie folgt ausgebildet sein.

Das Segment weist einen Steg zur Aufnahme einer Scharnieröffnung auf. Der Steg ist insbesondere stiftförmig ausgebildet und sitzt in einer Aussparung der Knochenplatte, so dass um den Steg herum genügend Platz ist, um eine Scharnieröffnung beweglich aufzunehmen. Weiter dient in einer Ausführungsform das Material des Segmentes an den Enden des Steges als Anschlag in einer axialen Richtung, also in Haupterstreckungsrichtung des Steges.

Bei einer Ausführungsform ist der Steg eines Segments einstückig ausgebildet, das heißt der Steg ist beispielsweise durch ein spanabhebendes Verfahren aus einem Vollmaterial gebildet.

Es ist insbesondere vorgesehen, dass alle Segmente der Osteosyntheseplatte einstückig ausgebildet sind. Dies erleichtert eine Herstellung aus biokompatiblem Material und vermeidet die Gefahr, dass sich von dem Osteosystem Komponenten lösen und unter Umständen im Körper des Patienten verbleiben könnten. Vorzugsweise sind die Segmente aus einem Vollmaterial wie Titan durch ein zerspanendes Verfahren hergestellt.

Bei einer alternativen Ausführungsform kann der Steg durch einen in das Segment eingesetzten Stift gebildet werden. Die Herstellung dieser Variante ist zumeist weniger aufwändig und der Steg kann aus einem anderen Material als das restliche Segment bestehen.

Die Scharniersegmente weisen eine Oberseite und eine Unterseite auf, wobei unter der Unterseite im Sinne der Erfindung die Seite verstanden wird, welche bei bestimmungsgemäßen Gebrauch dem Knochen zugewandt ist.

Die Unterseite ist, um sich der Kontur des Knochens annähern zu können, vorzugsweise konkav geformt. Die Oberseite kann konvex geformt sein.

Weiter kann das Segment zumindest zwei voneinander beabstandete Scharnieröffnungen aufweisen. So kann ein Segment an zumindest zwei Stegen des anderen Segments befestigt werden.

Dies ermöglicht eine stabilere mechanische Verbindung, da so die Stege kürzer ausgebildet sein können und dadurch die Bruchgefahr des Steges reduziert wird. Weiter wird bei klammerartig ausgebildeten Segmenten das Einrasten erleichtert.

Erfindungsgemäß ist das Segment U-förmig ausgebildet, wobei die hierdurch gebildete Ausnehmung einer oberen und einer unteren Hälfte auf einer geschlossenen Seite des U einen sich vergrößernden Federabschnitt aufweist.

Die Höhe der Ausnehmung zwischen den beiden Hälften des Segmentes steigt also am Ende des U-förmigen Abschnitts an. Dies ermöglicht, im Querschnitt gesehen, dass das Material von der oberen Hälfte ausgehend, sich um die Ecke herum nach unten erstreckt. Dieser die Erstreckungsrichtung ändernde Abschnitt ist aus dünnerem Material ausgebildet als die davor liegenden Abschnitte der unteren und oberen Hälfte und wirkt so, unter anderem auch aufgrund seiner Länge, als Federabschnitt.

Um beim Eindrehen einer Knochenschraube die Segmente festzulegen, ist ein Segment U-förmig ausgebildet und weist eine Durchführung auf, wobei die Durchführung auf einer unteren, zur Anlage an einen Knochen ausgebildeten Seite ein Gewinde aufweist.

Vorzugsweise verfügt eine gegenüberliegende obere Seite der Durchführung nicht über Mittel, die ausgebildet sind, um mit einem Gewinde der Knochenschraube in Eingriff zu kommen. Insbesondere ist die obere Seite der Durchführung glattwandig ausgebildet.

Eine korrespondierende Knochenschraube verfügt am Kopf über ein Gewinde sowie über eine über dem Gewinde liegende Anlagefläche, insbesondere über einen Konus oder eine sphärische Struktur.

Kommt nun das Kopfgewinde mit dem Gewinde des Segmentes in Eingriff, wird durch die darüber liegende glattwandige Struktur das U-förmig ausgebildete Segment zusammengedrückt und verklemmt sich auf dem Steg des gegenüberliegenden Segmentes.

Unter einem Gewinde wird jene Struktur verstanden, welche eine schraubenartige Verbindung ermöglicht. Es kann sich dabei auch um ein Gewinde handeln, welches durch die Schraube selbst eingeschnitten wird oder aus Gewindesegmenten besteht, insbesondere um den Winkel der einzubringenden Schraube zu variieren. So ist eine polyaxiale, winkelstabile Verbindung möglich.

Unter einer glattwandigen Struktur im Sinne der Erfindung wird eine Ausgestaltung verstanden, bei der es nicht zu einer Schraubverbindung kommt. Die glattwandige Struktur kann im einfachsten Fall als glatte Fläche ausgebildet sein, auf den ein Schraubenkopf zur Anlage kommt. Vorzugsweise ist die glattwandige Fläche aber als Konus, insbesondere als kegel- oder sphärenförmiger Konus, ausgebildet.

Durch die Erfindung kann ein Osteosynthesesystem bereitgestellt werden, welches eine vorstehend beschriebene Osteosyntheseplatte sowie eine Knochenschraube umfasst, welcher derart ausgebildet ist, dass durch das Eindrehen der Knochenschraube die Segmente der Osteosyntheseplatte gegeneinander festgelegt werden.

Das Osteosynthesesystem kann eine Knochenplatte oder ein Segment einer Knochenplatte umfassen. Die Knochenplatte oder das Segment ist zumindest abschnittsweise zweilagig ausgebildet und weist eine obere und eine untere Hälfte auf. Obere und untere Hälfte sind vorzugsweise federnd miteinander verbunden und somit zumindest in einem Ausgangszustand durch einen Spalt voneinander beabstandet. Das Segment beziehungsweise die Knochenplatte weist zumindest eine Durchführung auf, die sich durch die obere und die untere Hälfte erstreckt.

Durch die Durchführung kann eine Knochenschraube geführt werden.

Die Durchführung weist eine untere Hälfte auf, welche zumindest abschnittsweise ein Gewinde aufweist.

Unter einem Gewinde im Sinne der Erfindung wird jede Eingriffsstruktur verstanden, die dazu ausgebildet ist, mit einer korrespondierenden Struktur einer Knochenschraube eine formschlüssige Verbindung in einer axialen Richtung auszubilden.

Mithin kann das Gewinde beispielsweise auch als bloßer Steg ausgebildet sein.

Weiter ist denkbar, dass sich das Gewinde erst beim Eindrehen einer Schraube in das Material einschneidet.

Der minimale Durchmesser der Durchführung in der unteren Hälfte ist kleiner als der minimale Durchmesser der Durchführung in der oberen Hälfte.

Das Osteosynthesesystem weist des Weiteren eine Knochenschraube mit einem Gewinde auf, welches dazu ausgebildet ist in einen Knochen eingeschraubt zu werden.

Es kann sich insbesondere um ein Gewinde mit einer selbstschneidenden Spitze handeln.

Weiter weist die Knochenschraube einen Schraubenkopf auf.

Der Schraubenkopf weist eine Struktur zum Eingriff eines Handhabungswerkzeugs auf, mit welchem die Knochenschraube eingedreht wird.

Weiter weist der Schraubenkopf eine Sitzfläche auf. Die Sitzfläche ist ausgebildet, um neben oder in der oberen Durchführung zur Anlage zu kommen.

Im einfachsten Fall kann die Sitzfläche eine ebene Fläche sein, welche neben der Durchführung auf der Oberseite des Segmentes beziehungsweise der Knochenplatte zur Anlage kommt.

Vorzugsweise ist die Anlagefläche aber konisch, insbesondere sphärisch oder kegelförmig, ausgebildet und kommt an einer korrespondierenden konischen Fläche der oberen Durchführung zur Anlage.

Beim Eindrehen der Knochenschraube passiert zunächst das Kopfgewinde den oberen Teil der Durchführung.

Da der obere Teil der Durchführung einen größeren Durchmesser als das Kopfgewinde hat, kommt es nicht zu einer axialen formschlüssigen Verbindung.

Beim weiteren Eindrehen kommt das Kopfgewinde mit dem Gewinde der unteren Hälfte der Durchführung in Eingriff.

Sodann oder gleichzeitig kommt die Sitzfläche des Schraubenkopfes an der oberen Hälfte des Segmentes oder der Knochenplatte zur Anlage, so dass beim weiteren Eindrehen die beiden Hälften des Segmentes oder der Knochenplatte zusammengezogen werden.

Durch das aufeinander zu Bewegen der beiden Segmente können sich diese beispielsweise an einem anderen Segment festklemmen.

Denkbar ist aber auch ein anderes Bauteil, wie beispielsweise ein Cerclage-Kabel, zwischen den Segmenten festzuklemmen.

Das Osteosynthesesystem kann des Weiteren eine Bohrhülse umfassen, welche in die Durchführung einbringbar ist.

Derartige Bohrhülsen sind grundsätzlich bekannt. Diese werden verwendet, um vor dem Eindrehen der Knochenschraube in den Knochen mittels eines Bohrers einen Kanal in den Knochen einzubringen. Häufig sind derartige Bohrhülsen Teil einer Zielvorrichtung, mittels der vor dem Bohren genau ausgerichtet werden kann wo entlang sich der Kanal erstrecken soll.

Die Erfindung betrifft eine Bohrhülse, mit welcher die Hälften der Knochenplatte beziehungsweise des Segments aufeinander zubewegbar, also zusammenpressbar sind.

Hierzu kann die Bohrhülse ein Gewinde umfassen, welches in die untere Hälfte eingeschraubt wird.

Weiter kann über dem Gewinde eine Kontermutter angeordnet sein. Durch das Anziehen der Kontermutter werden die Hälften des Segments oder der Knochenplatte zusammengedrückt.

Es ist insbesondere so möglich, mittels der Bohrhülse ein erstes und ein zweites Segment winkelstabil gegeneinander festzulegen.

Das Osteosynthesesystem kann eine einschraubbare Bohrhülse umfassen.

Die Bohrhülse ist derart ausgebildet, dass das untere Ende der Bohrhülse beim Eindrehen am Knochen zu Anlage kommt und sich dort abstützt. So kann durch eine unterschiedliche Eindrehtiefe der Abstand des Segmentes oder der Knochenplatte zum Knochen eingestellt werden.

Im Fall der zuvor beschriebenen Osteosynteseplatte, die aus zumindest zwei Segmenten besteht, kann so auf einfache Weise der Winkel zwischen den Segmenten eingestellt werden.

Das Osteosynthesesystem kann des Weiteren eine Knochenschraube, umfassen.

Die Knochenschraube umfasst ein Gewinde zum Einschrauben in einen Knochen, ein Schraubenkopf mit einer Sitzfläche. Zwischen Gewinde zum Eindrehen in den Knochen und dem Schraubenkopf ist ein Kopfgewinde vorgesehen, dessen maximaler Durchmesser zwischen dem maximalen Durchmesser des Gewindes zum Eindrehen in den Knochen und dem Schraubenkopf liegt.

### Beschreibung der Zeichnungen

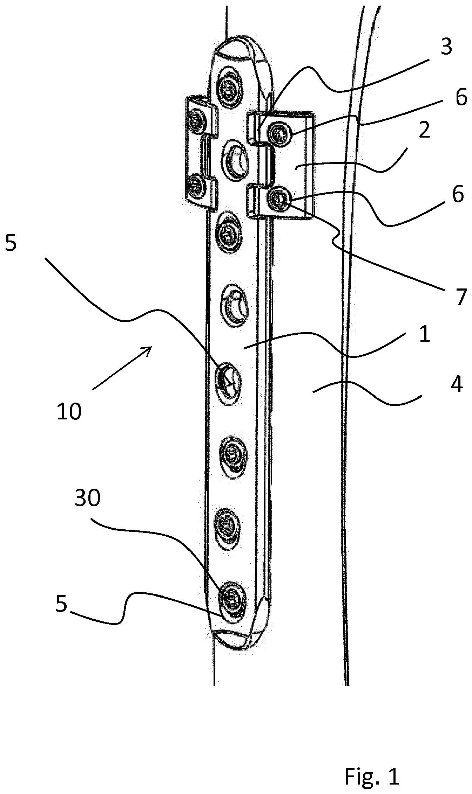

Der Gegenstand der Erfindung soll im Folgenden Bezug nehmend auf die Zeichnungen Fig. 1 bis Fig. 30 erläutert werden.

Fig. 1 zeigt eine schematische perspektivische Ansicht eines ersten Ausführungsbeispiels der Erfindung. Zu erkennen ist die an einem Knochen 4 angebrachte Osteosyntheseplatte 10, welche aus einem ersten Segment 1 und einem zweiten Segment 2 besteht.

Gegenüber dem zweiten Segment 2 ist noch ein weiteres Segment vorhanden.

Das erste Segment 1 ist im Wesentlichen als aus dem Stand der Technik bekannte Knochenplatte ausgebildet, welche eine Haupterstreckungsrichtung aufweist, entlang derer eine Mehrzahl von Durchführungen 5 angeordnet sind.

Bei den Durchführungen 5 handelt es sich bei diesem Ausführungsbespiel um Durchführungen, welche eine winkelstabile Verbindung mit Knochenschrauben 30 ermöglichen und welche darüber hinaus zur Kompression zum Schließen eines Frakturspaltes verwendet werden können. Ein derartiges System ist in der Offenlegungsschrift DE 10 2010 025001 A1 beschrieben.

Gemäß der Erfindung ist das zweite Segment 2 mittels der Scharniere 3 beweglich an dem ersten Segment 1 angeordnet.

Das zweite Segment 2 weist zwei Durchführungen 6 zum Einbringen von Knochenschrauben 7 auf und erstreckt sich quer zur Haupterstreckungsrichtung des ersten Segments.

Aufgrund der Verbindung durch die Scharniere 3 kann die durch die Segmente 1 und 2 gebildete Osteosyntheseplatte der runden Kontur des Knochens 4 folgen.

Weiter ist das Segment 2 gegenüber dem Segment 3 nur in einem Freiheitsgrad beweglich, nämlich schwenkbar über die Scharniere 3.

In axialer Richtung, also quer zu einer möglichen Bruchkante des Knochens 4, sind die Segmente 1 und 2 dagegen nicht beweglich.

Das flügelartig abstehende Segment 2 kann mithin als weiterer zusätzlicher Befestigungspunkt für eine Knochenschraube verwendet werden.

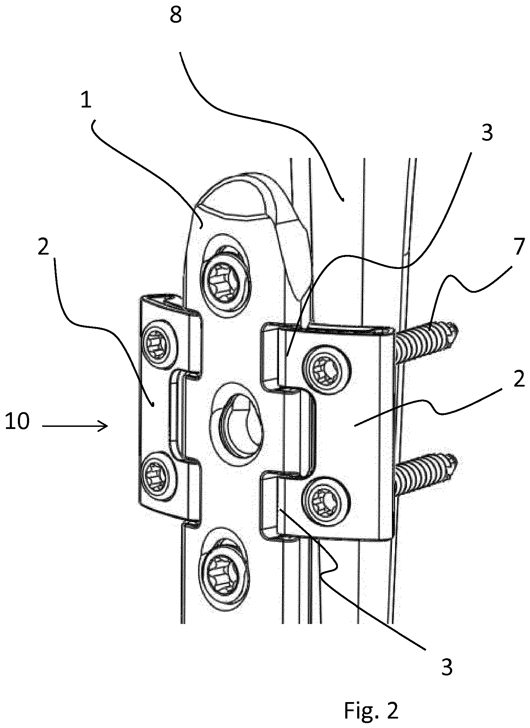

Bezug nehmend auf Fig. 2 soll die Funktion bei einer periprothetischen Osteosynthese erläutert werden.

Fig. 2 zeigt ausschnittsweise die in Fig. 1 dargestellte Osteosyntheseplatte 10 bestehend aus dem ersten Segment 1 und zwei weiteren Segmenten 2. Die über die Scharniere 3 flügelartig abstehenden Segmente 2 ermöglichen das Einbringen von Knochenschrauben 7, welche seitlich am Markkanal des Knochens und damit an einer Prothese 8 vorbei verlaufen.

Neben einer periprothetischen Versorgung können die zusätzlichen Segmente 2 auch verwendet werden, um zusätzliche Befestigungspunkte bei instabilem Knochenmaterial, insbesondere von älteren Patienten bereitstellen zu können.

Fig. 3 zeigt eine perspektivische Ansicht eines zweiten Segments 2.

Das Segment 2 ist U-förmig ausgebildet, weist eine Durchführung 5 auf, welche in einem oberen Bereich einen glattwandigen Abschnitt und im unteren Bereich einen Gewindeabschnitt 11 aufweist.

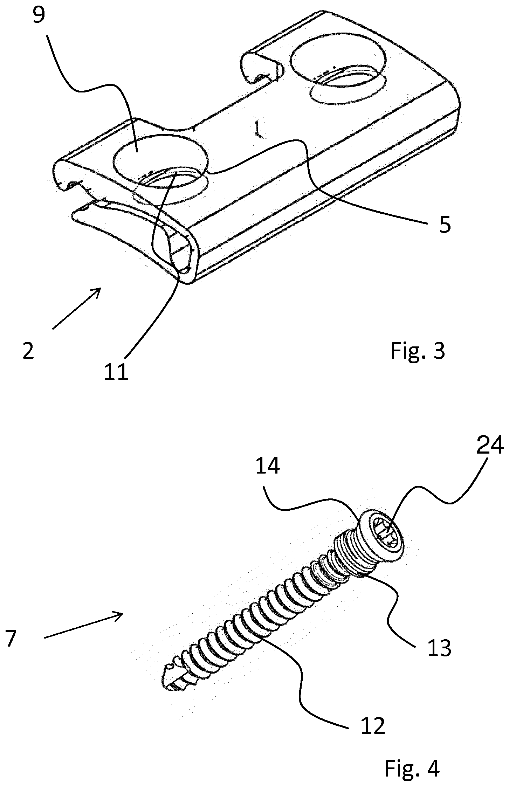

Das Segment 2 ist zum Zusammenwirken der in Fig. 4 dargestellten Knochenschraube ausgebildet.

Die Knochenschraube 7 umfasst ein Gewinde 12 zum Eindrehen in den Knochen sowie einen Schraubenkopf 24, welcher Mittel aufweist, um die Schraube mit einem Handhabungswerkzeug einzudrehen.

Weiter weist die Knochenschraube 7 oberhalb des Gewindes 12 ein Kopfgewinde 13 und eine darüber liegende Sitzfläche auf, welche als konischer Abschnitt 14 ausgebildet ist. Dieser kann insbesondere kegelförmig oder sphärisch ausgebildet sein kann.

Der Durchmesser des Kopfgewindes 13 liegt zwischen dem Durchmesser des Gewindes 12 und dem Durchmesser des Schraubenkopfes 24.

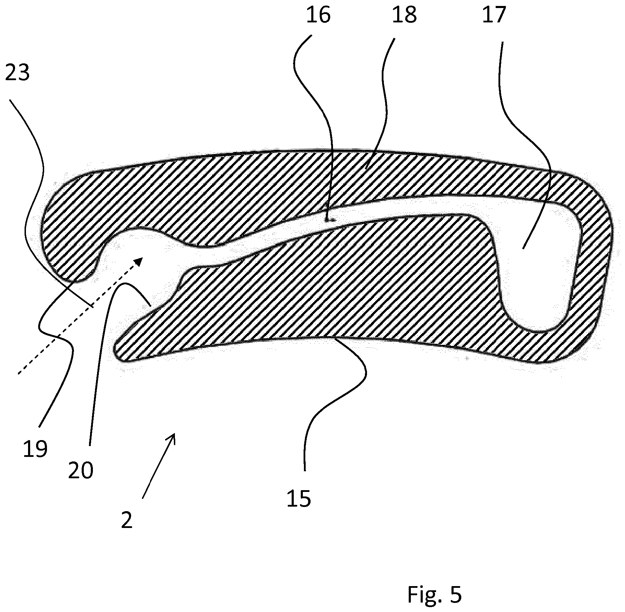

Fig. 5 zeigt eine Schnittansicht des in Fig. 3 dargestellten zweiten Segmentes.

Zu erkennen ist, dass das zweite Segment 2 U-förmig ausgebildet ist, also in eine untere Hälfte 15 und eine obere Hälfte 18 unterteilt ist.

Die untere Hälfte 15 ist auf der Unterseite konkav ausgebildet, um sich der Kontur des Knochens besser anzupassen.

Obere Hälfte 18 und untere Hälfte 15 werden durch eine Ausnehmung 16 voneinander getrennt.

Die Ausnehmung 16 weitet sich in einem vorderen Bereich, in welchem ein Scharnierflügel 19 und eine Einbuchtung 20 angeordnet ist, zunächst etwas auf, so dass dieser vordere Bereich klammerförmig ausgebildet ist, um auf einen stiftförmigen Steg eines anderen Segments (nicht dargestellt) aufgedrückt werden zu können.

Der Scharnierflügel 19 ist derart ausgebildet, dass er einen Steg zumindest abschnittsweise umschließen kann.

Um das werkzeuglose Aufrasten auf einen Steg zu erleichtern, sind sämtliche Kanten des vorderen Bereiches abgerundet.

Weiter ist der klammerartige Bereich der Ausnehmung 16 in Richtung der unteren Hälfte 15 geneigt, so dass die durch einen Pfeil 23 symbolisierte Einführrichtung schräg zur oberen und unteren Oberfläche des Segmentes 2 verläuft.

Hinter dem durch Scharnierflügel 19 und Einbuchtung 20 gebildeten klammerartigen Bereich verjüngt sich die Ausnehmung 16 und steigt in Richtung der oberen Hälfte 18 des Segments 2 an, so dass sich die obere Hälfte 18 in Richtung der geschlossenen Seite verjüngt.

In Richtung der geschlossenen Seite des Segments 2 weitet sich sodann die Ausnehmung 16 auf und bildet einen Biegeabschnitt 17, in welchem die obere Hälfte 18 nach unten abknickt, also die Richtung wechselt und sodann in die untere Hälfte übergeht, um insgesamt U-förmig ausgebildet zu sein.

Auf diese Weise ist das zweite Segment 2 als Klammer ausgebildet, bei welcher obere Hälfte 18 und untere Hälfte 15 gegeneinander federn können, so dass das zweite Segment 2 mit dem vorderen klammerartigen Abschnitt auf einem Steg einschnappen kann.

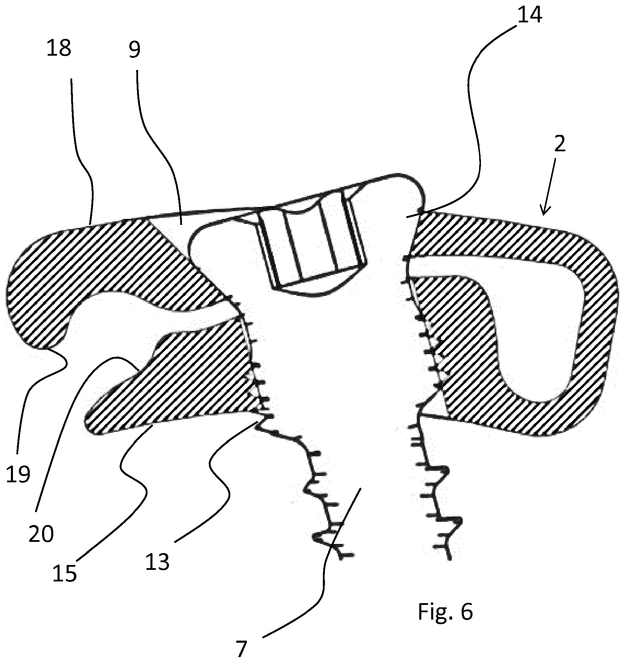

Fig. 6 zeigt eine weitere Schnittansicht des Segments 2, in welcher das Zusammenwirken mit der Knochenschraube 7 dargestellt ist.

Die in die Durchführung des Segments 2 eingebrachte Knochenschraube 7 weist ein Kopfgewinde 13 auf, welches mit einem Gewinde der Durchführung in der unteren Hälfte 15 korrespondiert.

Über dem Gewinde 13 weist die Schraube 7 einen konischen Abschnitt 14 auf, welcher einen größeren Durchmesser als das Gewinde aufweist.

Der konische Abschnitt 14 kommt an einem glattwandigen Abschnitt 9 der Durchführung in der oberen Hälfte 18 des zweiten Segments 2 zur Anlage.

Wird nun die Schraube weiter eingedreht, zieht diese die obere Hälfte 18 und die untere Hälfte 15 des zweiten Segments zusammen, so dass Scharnierflügel 19 und Einbuchtung 20 aufeinander zu bewegt werden.

Befindet sich zwischen Scharnierflügel und Einbuchtung ein Steg (nicht dargestellt), wird das zweite Segment auf diesem Steg kraftschlüssig befestigt, so dass die Segmente der Knochenplatte nicht gegeneinander beweglich sind.

Neben der Funktion des Festlegens auf einem korrespondieren Steg wird durch das Festlegen des klammerartig ausgebildeten Segments 2 auch erreicht, dass sich obere und untere Hälfte nicht gegeneinander aufweiten können und so das Segment vom Steg rutschen kann.

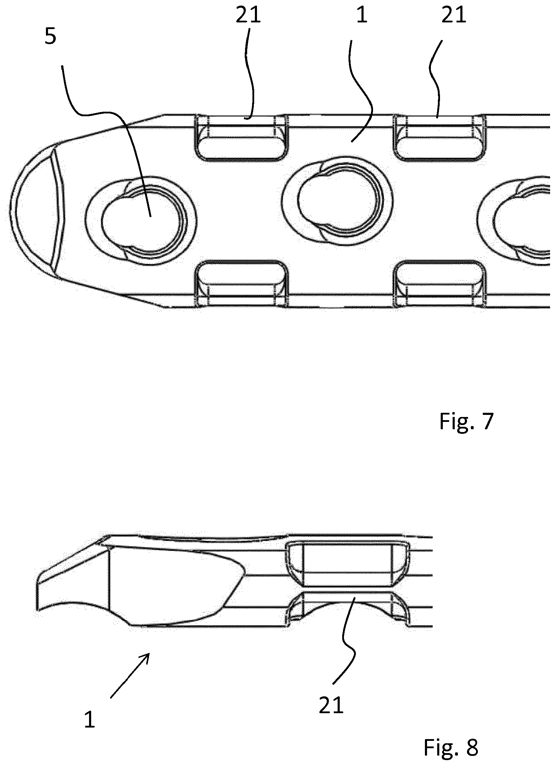

Fig. 7 zeigt eine Detaildarstellung eines ersten Segmentes 1, welches im Wesentlichen als Knochenplatte mit Durchführungen 5 ausgebildet ist.

An den Seiten umfasst die Knochenplatte mehrere Ausnehmungen, die mittels jeweils eines Steges 21 überspannt sind.

Der in Fig. 8 in der Seitenansicht zu sehende Steg 21 hat im Wesentlichen einen kreisförmigen Querschnitt und ist sowohl von der Oberseite als auch von der Unterseite der Knochenplatte beabstandet.

Wird nun ein zweites Segment (nicht dargestellt) angefügt, liegt dieses etwa auf derselben Höhe wie das erste Segment 1.

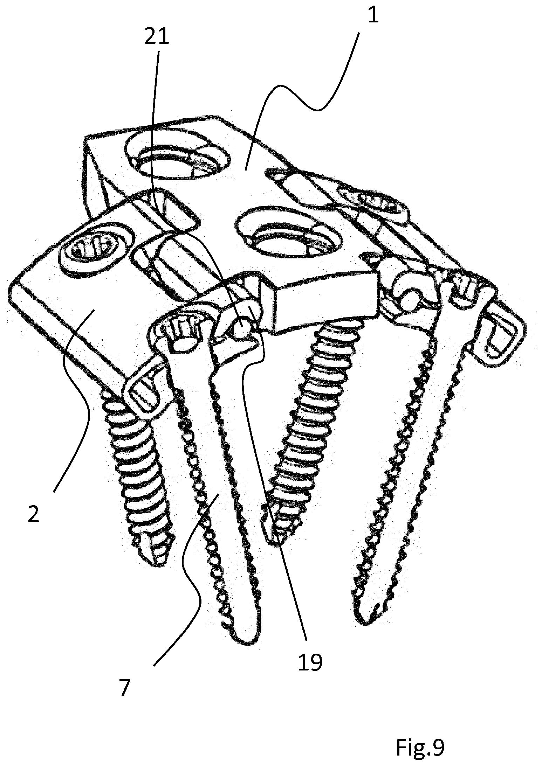

Fig. 9 zeigt eine perspektivische Schnittansicht einer Osteosyntheseplatte.

Zu erkennen ist das erste Segment 1. Das zweite Segment 2, welches klammerartig ausgebildet ist, ist auf den Steg 21 des ersten Segments 1 aufgeschoben.

Durch Eindrehen der Knochenschraube 7, welche ein Kopfgewinde umfasst, sind nunmehr erstes Segment 1 und zweites Segment 2 gegeneinander festgelegt.

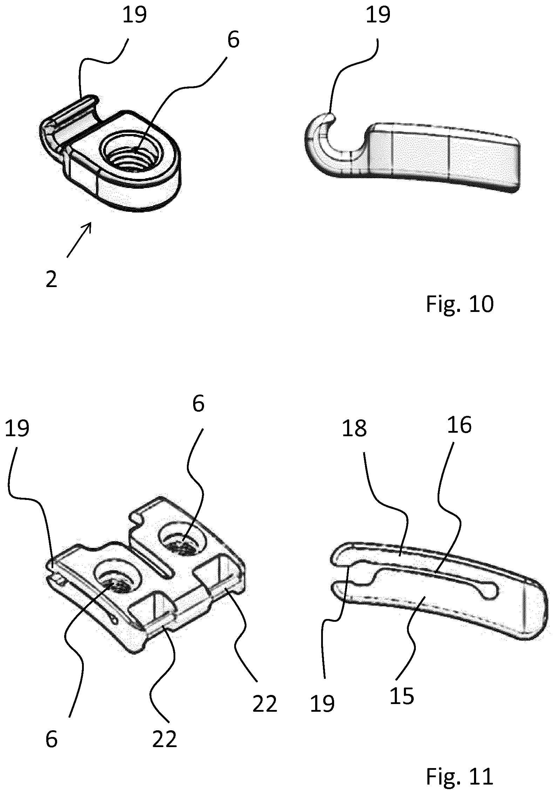

Fig. 10 zeigt eine alternative Ausführungsform eines zweiten Segments.

Das zweite Segment 2 umfasst eine Ausnehmung 6, in diesem Ausführungsbeispiel in Form eines Gewindes sowie einen hakenförmigen Scharnierflügel 19.

Mittels des hakenförmigen Scharnierflügels 19 kann das zweite Segment 2 auf die in den vorstehenden Zeichnungen dargestellte Knochenplatte aufgeschoben werden und hakt sich dort ein. Es kommt zu einer formschlüssigen Verbindung, welche allerdings gelöst werden kann, indem das zweite Segment nach oben geklappt und zur Seite gezogen wird.

In diesem Ausführungsbeispiel ist das zweite Segment 2 nur zum Eingriff in einen einzigen Steg ausgebildet.

Fig. 11 zeigt eine weitere Ausführungsform eines zweiten Segmentes. Dieses ist klammerartig ausgebildet und weist zwei Scharnierflügel 19 sowie zwei Durchführungen 6 auf, welche im Wesentlichen der in Fig. 3 dargestellten Ausführungsform entsprechen.

Im Unterschied zu den in Fig. 3 dargestellten Ausführungsform handelt es sich insofern um ein Doppelscharnier, bei dem das zweite Segment auf der dem Scharnierflügel gegenüberliegenden Seite zwei Stege 22 aufweist, an denen ein weiteres Segment schwenkbar angebracht werden kann.

In der rechts dargestellten Schnittansicht gut zu erkennen ist der Scharnierflügel 19, welcher einen klammerartigen Abschnitt bildet, sowie dass das Segment in eine untere Hälfte und eine obere Hälfte 18 unterteilt ist, die durch die Ausnehmung 16 voneinander getrennt sind. Das Segment ist mithin U-förmig ausgebildet.

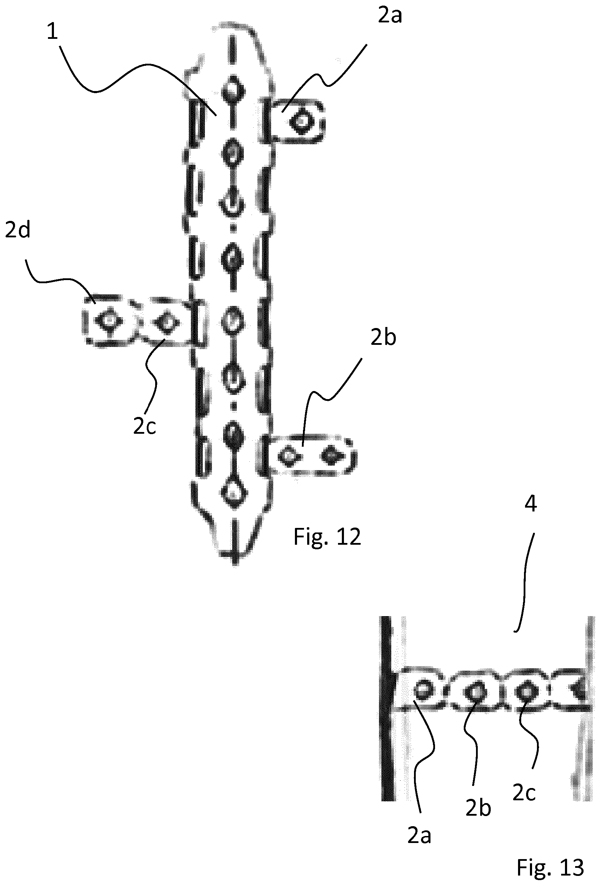

Fig. 12 zeigt schematisch ein als Knochenplatte ausgebildetes erstes Segment 1, welches im Wesentlichen über seine gesamte Länge voneinander beabstandete Stege aufweist, an denen Segmente 2a, 2b, 2c, 2d angebracht werden können.

Zu erkennen ist, dass die Segmente 2a bis 2d an unterschiedlichen Stellen des ersten Segmentes 1 angebracht werden können.

Weiter können je nach Indikation verschiedene Segmente angebracht werden. So ist das Segment 2a kürzer und weist nur eine Durchführung auf. Das Segment 2b ist länger ausgebildet und umfasst zwei voneinander beabstandete Durchführungen.

Die Segmente 2c und 2d bilden mit dem Segment 1 eine Gelenkkette.

Wie in Fig. 13 dargestellt, können die Segmente 2a bis 2c eine Kette bilden, mit der ein Knochen 4 ganz oder teilweise umschlungen werden kann.

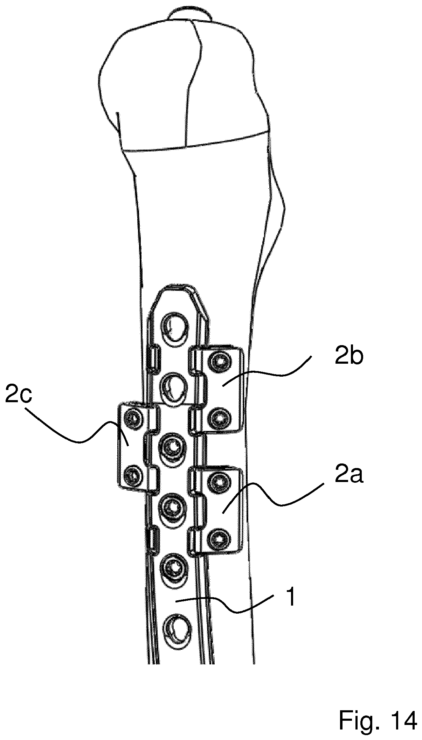

Fig. 14 zeigt eine weitere Ausführungsform der Erfindung, eine auf einem Knochen aufliegende Osteosyntheseplatte, welche aus dem Segment 1, welches als länglich ausgebildete Knochenplatte mit mehreren Durchführungen ausgebildet ist, und den seitlich angehängten Segmenten 2a bis 2c besteht.

Im Unterschied zu der in Fig. 1 dargestellten Ausführungsvariante ist das Segment 2c gegenüber den Segmenten 2a und 2b versetzt angeordnet.

Hierzu umfasst das erste Segment eine Vielzahl von Stellen an welchen weitere Segmente angesetzt werden können.

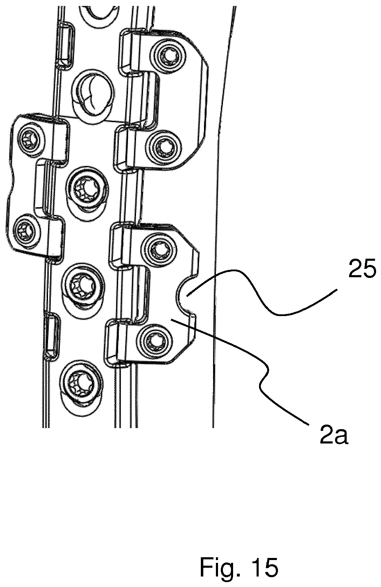

Fig. 15 zeigt eine weitere Ausführungsform, welche im Wesentlichen Fig. 14 entspricht.

Im Unterschied zu Fig. 14 weist das Segment 2a eine seitliche Ausnehmen 25 auf, welche beispielsweise als Führungsnut für ein Cerclage-Kabel dienen kann.

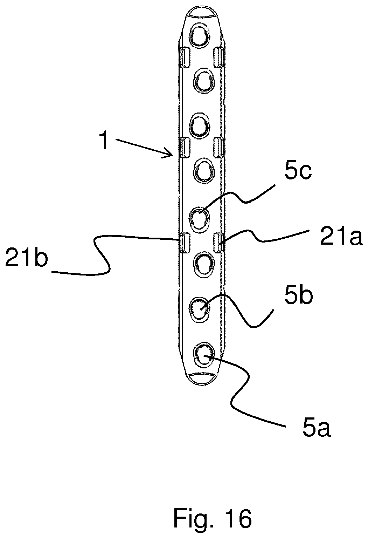

Fig. 16 zeigt eine weitere Variante eines ersten Segmentes 1.

Das Segment 1 weist mehrere, sich gegenüberstehende Stege 21a, 21b auf, an denen weitere Segmente angesetzt werden können.

Weiter weist das erste Segment 1 eine Vielzahl von Durchführungen 5a bis 5c auf.

Diese sind quer zur Haupterstreckungsrichtung des ersten Segments jeweils zueinander versetzt.

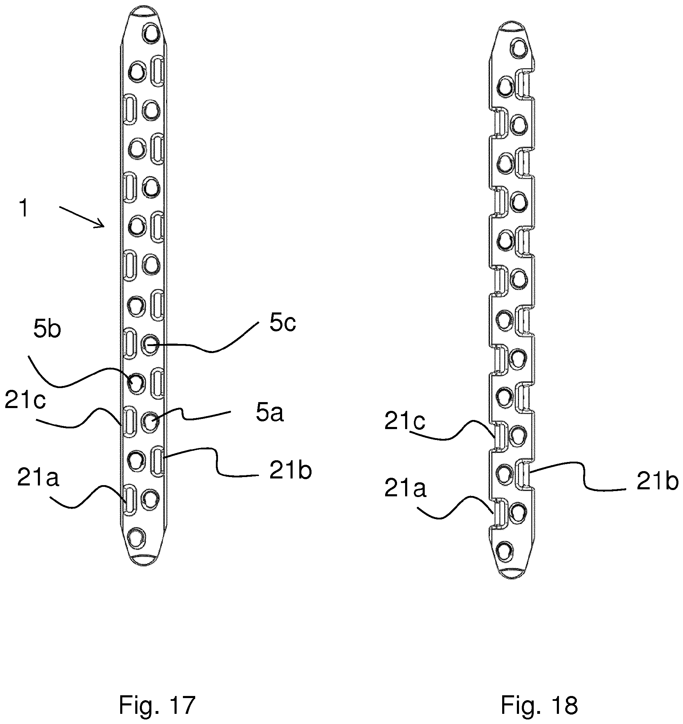

Fig. 17 zeigt eine weitere Ausführungsvariante eines ersten Segmentes 1.

Die Durchführungen 5a bis 5c sitzen jeweils im Wechsel quer zur Haupterstreckungsrichtung in eine rechte und linke Richtung versetzt zueinander.

Jeweils gegenüber einer Durchführung ist ein Steg 21a bis 21c zum Ansetzen eines zweiten Segments angeordnet. Die Stege 21a bis 21c sind mithin jeweils ebenfalls nach Rechts und Links, also quer zur Haupterstreckungsrichtung, versetzt.

Diese Ausführungsform ermöglicht eine besonders schmale Ausgestaltung eines ersten Segmentes 1.

Die in Fig. 18 dargestellte Ausführungsvariante entspricht im Wesentlichen Fig. 17, mit dem Unterschied, dass die Stege 21a bis 21c nicht genau Randseitig, sondern zur Mitte des ersten Segmentes 1 versetzt angeordnet sind. So wandert der Drehpunkt in Richtung der Mitte der Platte, was eine noch schmalere Ausgestaltung bei angesetzten seitlichen weiteren Segmenten ermöglicht.

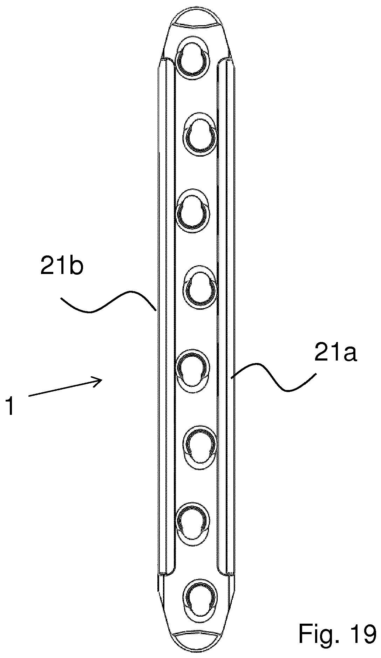

Fig. 19 zeigt eine weitere Ausführungsform eines ersten Segmentes.

Bei dieser Ausführungsvariante sind ebenfalls seitlich sich gegenüberliegende Stege 21a und 21b angeordnet, welche einen Großteil der Länge der Knochenplatte einnehmen.

Segmente die an diesem ersten Segment angehängt werden können (nicht dargestellt) können daher in axialen Richtung der Stege 21a, 21b verschoben werden.

Daher eignet sich diese Ausführungsform besonders in Verbindung mit den zuvor dargestellten festlegbaren Segmenten.

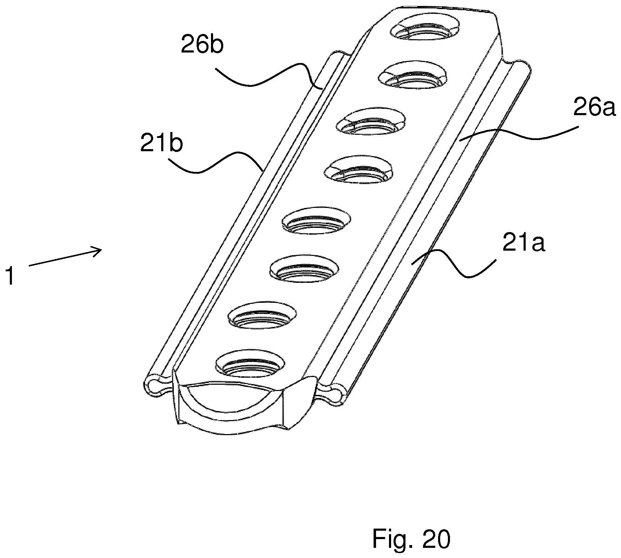

Fig. 20 zeigt eine weitere Ausführungsform eines ersten Segmentes.

Bei dieser Ausführungsvariante sind ebenfalls seitlich sich gegenüberliegende Stege 21a und 21b angeordnet, welche einen Großteil der Länge der Knochenplatte einnehmen.

Unter anderem um eine höhere Stabilität zu erreichen, ist der Bereich zwischen den randseitigen Stegen 21a und 21b zwischen Segment nicht ausgespart sondern als Schiene 26a, 26b ausgebildet.

Hierdurch wird zwar die Verstellmöglichkeit des Winkels möglicherweise eingeschränkt, das heißt, das zweite Segment (nicht dargestellt) lässt sich in einem kleineren Winkelbereich verstellen.

Mit einem derartigen schienenartigen System ist es neben einer erhöhten Stabilität auch auf einfache Weise möglich, dass das zweite Segment nicht klammerförmig aufgerastet wird sondern in Richtung der Haupterstreckungsrichtung des ersten Segmentes aufgeschoben wird.

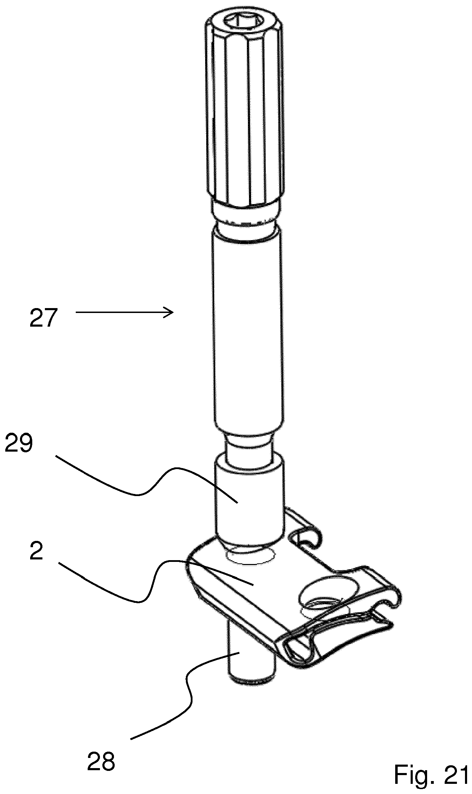

Bezugnehmend auf Fig. 21 soll erläutert werden, wie mittels einer Bohrhülse eine winkelstabile Festlegung von zwei Segmenten gegeneinander möglich ist.

Zu erkennen ist ein Segment 2, welches im Wesentlichen dem in Fig. 3 dargestellten Segment entspricht.

Ein weiteres Segment, an welchem das Segment 2 befestigt sein soll, ist zur besseren Übersicht nicht dargestellt.

In eine Durchführung des Segments 2 ist eine Bohrhülse 27 eingeschraubt.

Hierzu umfasst die Bohrhülse ein Gewinde 28, welches in das Gewinde der unteren Hälfte des Segments 2 einschraubbar ist.

Ansonsten umfasst die Bohrhülse einen mittigen Kanal und dient als Zielvorrichtung für einen Bohrer.

Die Bohrhülse umfasst des Weiteren eine Kontermutter 29, welche auf dem Gewinde 28 sitzt.

Bei Erreichen eines gewünschten Winkels kann die Kontermutter 29 angezogen werden. Diese drückt nun auf die obere Oberfläche des Segments 2 und schiebt die beiden Hälften des Segmentes 2 zusammen, so dass sich dieses winkelstabil an einem weiteren Segment festklammert.

Bei einer anderen Variante, bei der der Winkel nicht über die Einschraubtiefe der Bohrhülse eingestellt wird, ist auch denkbar, dass unmittelbar über das Gewinde der Bohrhülse die Hälften des Segmentes zusammengezogen werden. Die hier dargestellte Kontermutter 29 wäre dann nicht als Mutter, sondern gegenüber dem Gewinde verdickter Abschnitt der Bohrhülse ausgebildet.

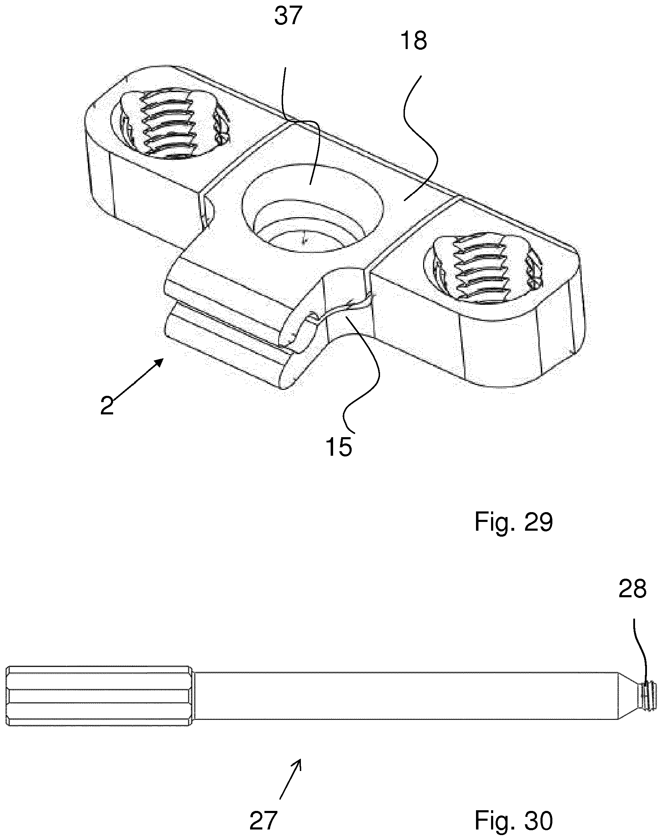

Eine derartige Ausführungsform ist in Fig. 30 gezeigt. Die Bohrhülse 27 weist ein Gewinde 28 zum Einschrauben in ein Segment auf. Beim Einschrauben der Bohrhülse 27 kommt eine oberhalb des Gewindes 28 angeordnete Anlagefläche, die in dieses Ausführungsbeispiel konisch ausgebildet ist, an der Oberseite des Segments zur Anlage und zieht die Hälften des Segments zusammen (nicht dargestellt).

Bei der in Fig. 21 dargestellten Ausführungsform kann dagegen der Winkel kann zunächst dadurch eingestellt werden, dass bei einer unterschiedlichen Einschraubtiefe das untere Ende der Bohrhülse unterschiedlich tief unten aus dem Segment 2 hervorsteht.

Das untere Ende der Bohrhülse kommt am Knochen (nicht dargestellt) zur Anlage, so dass über die Einschraubtiefe der Abstand des Segments 2 zum Knochen und damit der Winkel gegenüber einem anderen Segment (nicht dargestellt) eingestellt werden kann.

Die in Fig. 21 und 30 dargestellten Bohrhülsen ermöglichen eine winkelstabile Verbindung bereits beim Einbringen einer Bohrung in den Knochen.

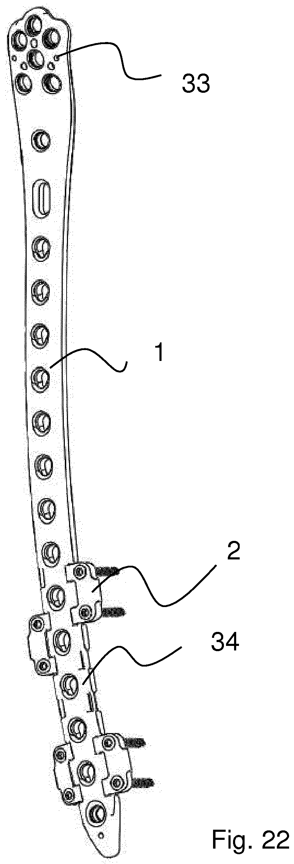

Fig. 22 zeigt eine weitere Ausführungsform der Erfindung.

Zu erkennen ist ein erstes Segment 1, welches als längliche Knochenplatte ausgebildet ist.

Das als Knochenplatte ausgebildete erste Segment 1 ist, um sich der Form des Knochens anzupassen, in diesem Ausführungsbeispiel sowohl in einer zum Knochen parallel verlaufenden Ebene als auch in einer senkrecht verlaufenden Ebene gebogen.

Weiter weist das erste Segment 1 einen verbreiterten Kopf 33 auf, welcher der Anbindung an das dickere Ende des Knochens dient.

Gegenüber dem darunter liegenden Stück für den Schaft des Knochens können im Kopf 33 mehrere Durchführungen für Knochenschrauben nebeneinander angeordnet sein.

In einem unteren Bereich 42 sind eine Mehrzahl von Stegen vorgesehen, die der Befestigung von zweiten Segmenten 2 dienen, wie dies bereits in den vorangegangenen Zeichnungen dargestellt wurde.

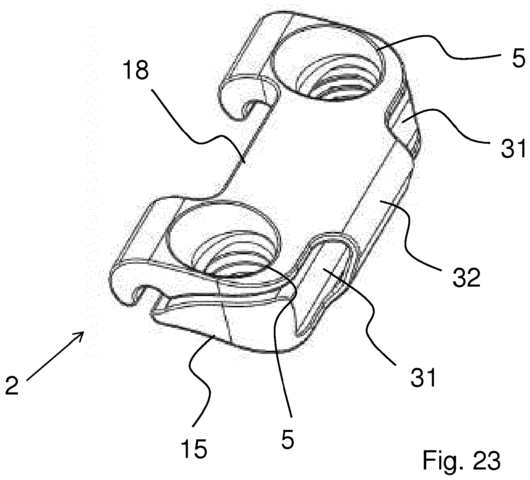

Fig. 23 zeigt eine weitere Ausführungsvariante eines Segments 2.

Dieses ist zur Befestigung an einer Knochenplatte ausgebildet und umfasst seinerseits zwei Durchführungen 5 für Knochenschrauben.

Im Unterschied zu dem in Fig. 2 dargestellten Segment weist der Verbindungsteil 32, welcher die obere Hälfte 18 mit der unteren Hälfte verbindet, randseitig zwei Ausnehmungen 31 auf.

Aufgrund der Ausnehmungen 31 ist der Verbindungsteil 32 geschwächt, so dass gegenüber einer Ausführung ohne Ausnehmungen weniger Kraft aufgewandt werden muss, um obere Hälfte 18 und untere Hälfte 19 auseinanderzudrücken.

Es versteht sich, dass ein derartiger Effekt beispielsweise auch dadurch erzielt werden kann, dass der Verbindungsteil 32 dünner ausgebildet ist, als obere und untere Hälfte. Die Ausnehmungen 31 und die damit verbundene mögliche dickere Ausgestaltung des Verbindungsteils 32 ermöglicht aber eine leichtere Herstellung, insbesondere wenn das Segment 2 aus Vollmaterial hergestellt wird.

Weiter ist das Segment 2 so robuster.

Es versteht sich, dass insbesondere bei dieser Ausführungsform eine Verformung beim Auseinanderdrücken von oberer Hälfte 18 und unterer Hälfte 15 vor allem in dem Verbindungsbereich 32 stattfindet, wohingegen obere Hälfte 18 und untere Hälfte 15 ansonsten dicker ausgebildet sind als der Verbindungsteil 32 und daher nicht oder nur wenig zur Federwirkung beitragen.

Obere Hälfte 18 und untere Hälfte 15 sind mithin insbesondere im Bereich der Durchführungen 5 dick und damit robust ausgebildet.

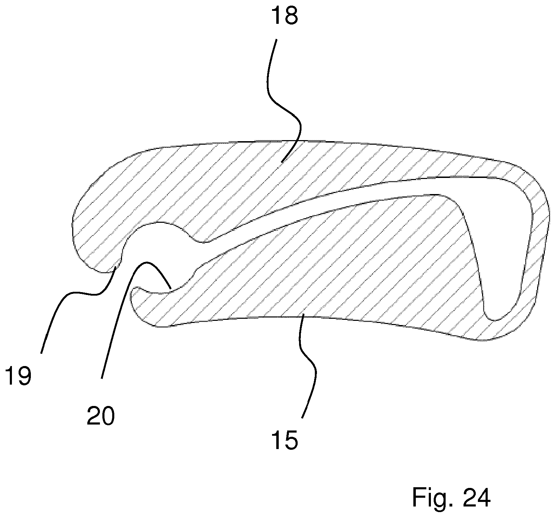

Fig. 24 zeigt eine Schnittansicht der in Fig. 23 dargestellten Ausführungsvariante eines zweiten Segments.

Gegenüber der in Fig. 5 dargestellten Ausführungsvariante ist die Einbuchung 20 in der unteren Hälfte 15 stärker ausgeprägt.

Der gegenüberliegende Scharnierflügel 19 sowie die Ausbuchtung 20 bildet zwischen der oberen Hälfte 18 und der unteren Hälfte 15 einen im wesentlichen runden Bereich, in welchem im aufgerasteten Zustand ein Steg des ersten Segments angeordnet ist.

Auch dieses Segment wird schräg von oben auf das vorzugsweise als Knochenplatte ausgebildete andere Segment aufgerastet.

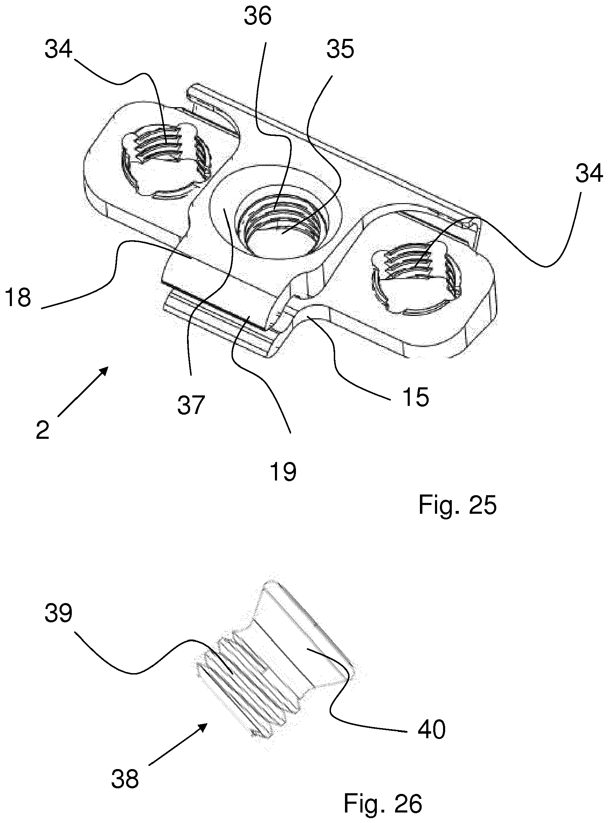

Fig. 25 zeigt eine weitere alternative Ausführungsvariante eines zweiten Segments 2.

Bei dieser Ausführungsvariante sind Verriegeln des Segments und Einbringen von Knochenschrauben voneinander entkoppelt.

Auch dieses Segment 2 ist klammerförmig ausgebildet und weist eine untere Hälfte 15 und eine obere Hälfte 18 auf.

In dieser Ausführungsvariante befinden sich nur in der unteren Hälfte Gewinde 34 zum Einbringen von Knochenschrauben. Statt eines Gewindes kann auch eine Durchführung ohne Gewindegänge vorhanden sein.

Weiter umfasst das Segment 2 eine weitere Durchführung 35, welche dem Verriegeln des Segments 2 dient.

Die Durchführung 35 umfasst ein Gewinde 36, welches in der unteren Hälfte 15 angeordnet ist, sowie eine Sitzfläche 37 in der oberen Hälfte 18.

Die Sitzfläche 37 ist in diesem Ausführungsbeispiel konisch ausgebildet. Es ist aber denkbar, dass beispielsweise auch einfach die Oberseite der oberen Hälfte 18 als Sitzfläche dient.

Das als Klammer ausgebildete Segment kann auf einen Steg einer Knochenplatte aufgerastet werden und kann im Anschluss bereits vor dem Einsetzen verriegelt werden.

Hierzu kann die in Fig. 26 dargestellte Schraube 28 verwendet werden.

Diese ist nicht als Knochenschraube ausgebildet, besitzt also kein Gewinde zum Einschrauben in den Knochen.

Das Gewinde 39 ist dagegen ausgebildet, um in das Gewinde der unteren Hälfte des in Fig. 25 dargestellten Segments eingedreht zu werden. Die Sitzfläche 40 am Schraubenkopf kommt an der Sitzfläche des in Fig. 25 dargestellten Segments zur Anlage und zieht beim Eindrehen obere und untere Hälfte des Segments zusammen, so dass dieses auf dem Steg einer Knochenplatte verriegelt wird.

Das in Fig. 25 dargestellte Segment hat einen mittig angeordneten Scharnierflügel 19.

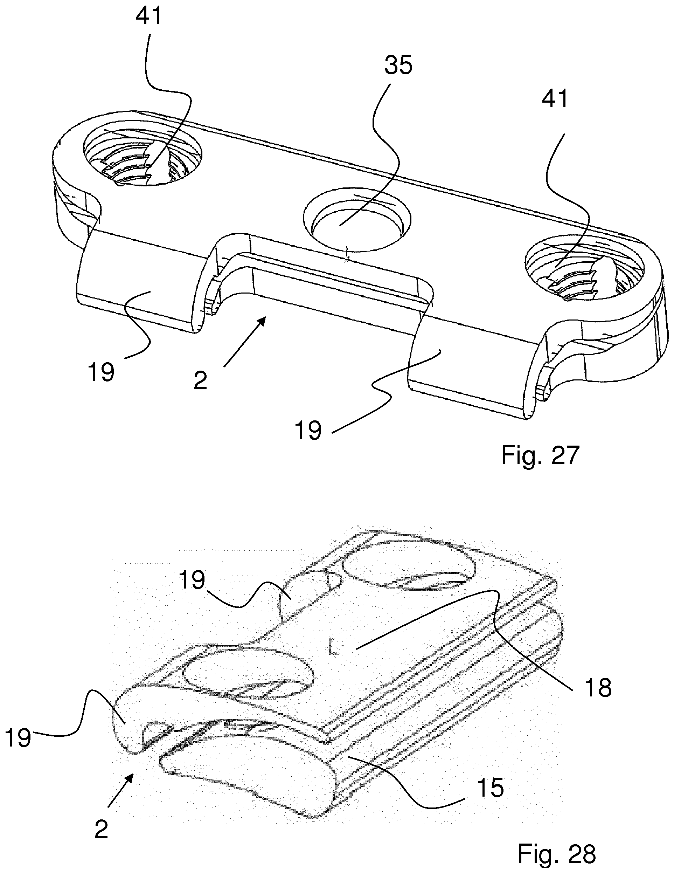

Fig. 27 zeigt eine weitere Ausführungsvariante, bei welcher eine Verriegelung des Segments 2 bereits vor dem Einsetzen möglich ist.

Wie die in Fig. 25 dargestellte Ausführungsvariante umfasst das Segment 2 eine Durchführung 35, welche der Ausführungsvariante in Fig. 25 entspricht und mit welcher das Segment 2 bereits vor dem Einsetzen und Eindrehen von Knochenschrauben verriegelt werden kann.

Die Durchführungen 41 umfassen in diesem Ausführungsbeispiel jeweils ein Gewinde und dienen dem Eindringen einer Knochenschraube.

Denkbar ist, die Durchführungen 41 zusätzlich zum Verriegeln zu verwenden, dass also wenn eine Knochenschraube eingedreht wird, obere und untere Hälfte weiter zusammengezogen werden und so die Verriegelung verstärkt wird. Dies ist aber nicht notwendig.

Das Segment 2 umfasst in diesem Ausführungsbeispiel zwei voneinander beabstandete Scharnierflügel 19, zwischen denen die Durchführung 35 zum Einsetzen einer Schraube sitzt, die ausschließlich der Verriegelung dient.

Fig. 28 zeigt eine weitere Ausführungsform eines Segments.

Im Unterschied zu den zuvor dargestellten Segmenten ist dieses Segment nicht als Klammer ausgebildet, kann also nicht aufgerastet werden, sondern obere und untere Hälfte 18, 15 sind separate Teile.

Ansonsten ähnelt das Segment von seiner Ausgestaltung her dem in Fig. 3 dargestellten Segment.

Das Segment 2 umfasst zwei voneinander beabstandete Scharnierflügel 19.

Fig. 29 zeigt eine weitere Ausführungsform, bei welcher das Segment 2 nicht als Klammer ausgebildet ist.

Bei dieser Ausführungsvariante sind ähnlich wie in der Ausführungsvariante gemäß Fig. 25 Verriegelung und Einbringen von Knochenschrauben voneinander entkoppelt.

Das Segment 2 umfaßt ein mittig eingesetztes Oberteil 18, welches eine Sitzfläche 37 aufweist und welches ausschließlich der Verriegelung des Segments 2 auf einer Knochenplatte dient.

Angrenzend zum Oberteil 18 ist das Unterteil 15 in diesem Ausführungsbeispiel verdickt ausgebildet, so dass das Unterteil randseitig in etwa die gleiche Höhe hat wie im mittleren Bereich, in welchem das Oberteil 18 eingesetzt ist.

Die Erfindung ermöglicht auf sehr einfache Weise die Ausbildung einer Osteosyntheseplatte für flexible Einsatzzwecke. Insbesondere kann die Osteosynthese von periprothetischen Frakturen verbessert werden.

### Bezugszeichenliste

- 1: erstes Segment
- 2: zweites Segment
- 3: Scharnier
- 4: Knochen
- 5: Durchführung
- 6: Durchführung
- 7: Knochenschraube
- 8: Femurprothese
- 9: glattwandiger Abschnitt
- 10: Osteosyntheseplatte
- 11: Gewindeabschnitt
- 12: Gewinde
- 13: Kopfgewinde
- 14: konischer Abschnitt
- 15: untere Hälfte
- 16: Ausnehmung
- 17: Biegeabschnitt
- 18: obere Hälfte
- 19: Scharnierflügel
- 20: Einbuchtung
- 21: Steg
- 22: Steg
- 23: Pfeil
- 24: Schraubenkopf
- 25: Ausnehmung
- 26: Schiene
- 27: Bohrhülse
- 28: Gewinde
- 29: Kontermutter
- 30: Knochenschraube
- 31: Ausnehmung
- 32: Verbindungsteil
- 33: verbreiterter Kopf
- 34: Gewinde
- 35: Durchführung
- 36: Gewinde
- 37: Sitzfläche
- 38: Schraube
- 39: Gewinde
- 40: Sitzfläche
- 41: Durchführung
- 42: unterer Bereich

## Patentansprüche

1. Osteosyntheseplatte, umfassend ein erstes Segment (1), welches als Knochenplatte mit einer Mehrzahl von Durchführungen (5) für Knochenschrauben (30) ausgebildet ist, sowie zumindest ein zweites Segment (2), welches U-förmig ausgebildet ist und zumindest eine weitere Durchführung (6) für eine Knochenschraube (7) aufweist, **dadurch gekennzeichnet, dass** das zweite Segment (2) lösbar mit dem ersten Segment (1) verbindbar ist und der Winkel, in welchem erstes und zweites Segment zueinander angeordnet sind, veränderbar ist, wobei erstes und zweites Segment klammerartig ineinander einrastbar sind, wobei das zweite Segment (2) eine obere Hälfte (18) und eine untere Hälfte (15) aufweist und durch ein Aufeinanderzubewegen der oberen und unteren Hälfte auf einem Steg (21) des ersten Segments (1) kraftschlüssig befestigbar ist,
und wobei erstes und zweites Segment (1,2) durch Einbringen einer Schraube (7) in das Gewinde einer Durchführung (6) des zweiten Segmentes (2) gegeneinander festlegbar sind.

2. Osteosyntheseplatte nach dem vorstehenden Anspruch, **dadurch gekennzeichnet, dass** das zweite Segment (2) als Klammer ausgebildet ist, insbesondere dass das zweite Segment (2) U-förmig ausgebildet ist.

3. Osteosyntheseplatte nach einem der vorstehenden Ansprüche, **dadurch gekennzeichnet, dass** das zweite Segment (2) schwenkbar am ersten Segment (1) angeordnet ist.

4. Osteosyntheseplatte nach dem vorstehenden Anspruch, **dadurch gekennzeichnet, dass** erstes und zweites Segment mittels eines Scharniers (3) beweglich miteinander verbunden sind.

5. Osteosyntheseplatte nach einem der vorstehenden Ansprüche, **dadurch gekennzeichnet, dass** erstes und zweites Segment werkzeuglos trenn- und/oder verbindbar ausgebildet sind.

6. Osteosyntheseplatte nach einem der vorstehenden Ansprüche, **dadurch gekennzeichnet, dass** das erste Segment (1) eine Mehrzahl von Stegen (21) zur Aufnahme mindestens eines Scharnierflügels (19) aufweist.

7. Osteosyntheseplatte nach einem der vorstehenden Ansprüche, **dadurch gekennzeichnet, dass** eine Vielzahl von Segmenten (1,2) aneinander anreihbar sind.

8. Osteosyntheseplatte nach dem vorstehenden Anspruch, **dadurch gekennzeichnet, dass** das zweite Segment (2) sowohl einen Steg (22) zur Aufnahme eines Scharnierflügels (19) als auch eine Scharnieröffnung aufweist.

9. Osteosyntheseplatte nach einem der vorstehenden Ansprüche, **dadurch gekennzeichnet, dass** zumindest eine Durchführung des ersten und/oder zweiten Segmentes als Gewinde, Gewindesegment oder verformbaren Rippe ausgebildet ist und/oder dass erstes und zweites Segment durch Einbringen einer Knochenschraube in eine Durchführung des zweiten Segmentes gegeneinander festlegbar sind.

10. Segment (2) für eine Osteosyntheseplatte, umfassend einen Scharnierflügel (19) zum Eingriff in einen Steg (21) eines weiteren Segments sowie zumindest eine Durchführung (6) zur Aufnahme einer Knochenschraube, wobei das Segment (2) U-förmig ausgebildet ist und eine Durchführung (6) aufweist, wobei die Durchführung auf einer unteren zur Anlage an einen Knochen ausgebildeten Seite ein Gewinde (11) aufweist, wobei das Segment (2) U-förmig ausgebildet ist, wobei die zwei Hälften (15,18) der U-förmigen Ausgestaltung wie eine Feder wirken, so dass ein klammerartig ausgebildeter Scharnierflügel es zulässt, dass das Segment (2) aufgeschoben werden kann, indem sich die U-förmige Ausgestaltung aufweitet und sodann einrastet das Segment eine obere und eine untere Hälfe (15,18) aufweist, und wobei sich die Durchführung (22) durch die obere und die untere Hälfte (15,18) erstreckt und wobei die Durchführung (22) in der unteren Hälfte (15) zumindest abschnittsweise ein Gewinde aufweist.

11. Segment (2) für eine Osteosyntheseplatte nach einem der vorstehenden Ansprüche, **dadurch gekennzeichnet, dass** ein Segment einen Steg (22) zur Aufnahme eines Scharnierflügels aufweist.

12. Segment (2) für eine Osteosyntheseplatte nach einem der vorstehenden Ansprüche, **dadurch gekennzeichnet, dass** eine zur Anlage an einen Knochen ausgebildete Unterseite konkav geformt ausgebildet ist,
und/oder dass das Segment zumindest zwei voneinander beabstandete Scharnierflügel (19) aufweist,
und/oder dass das Segment (2) U-förmig ausgebildet ist, wobei die dadurch gebildete Ausnehmung zwischen einer oberen und einer unteren Hälfte (18,15) auf der geschlossenen Seite einen sich vergrößernden Federabschnitt aufweist,
und/oder dass das Segment (2) auf einer gegenüberliegenden oberen Seite glattwandig (9) ausgebildet ist,
und/oder dass das Segment einen U-förmigen Querschnitt aufweist, wobei die offene Seite als Scharnierflügel (19) ausgebildet ist,
und/oder dass das Segment einstückig ausgebildet ist,
und/oder dass das Segment aus Metall, insbesondere aus Titan, einer Titanlegierung oder Edelstahl, besteht,
und/oder dass das Segment als Klammer ausgebildet ist.

## Claims

1. An osteosynthesis plate, comprising a first segment (1) in form of a bone plate that has a plurality of passages (5) for bone screws (30), and at least a second segment (2), which is U-shaped and which has at least one further passage (6) for a bone screw (7), **characterized in that** the second segment (2) is releasably connectable to the first segment (1), and wherein the angle in which the first and second segments are arranged relative to one another can be modified, wherein the first and the second segments can be snapped clip-like together, wherein the second segment (2) has an upper half (18) and a lower half (15) and can be fastened on a web (21) of the first segment (1) with a force-locked connection by moving the upper and lower halves toward each other,
and wherein the first and second segments (1,2) can be fixed to each other by introducing a screw (7) into a passage (6) of the second segment (2).

2. The osteosynthesis plate as claimed in the preceding claim, wherein the second segment (2) has a clip-like configuration, in particular wherein the second segment (2) is U-shaped.

3. The osteosynthesis plate as claimed in any of the preceding claims, wherein the second segment (2) is pivotally mounted to the first segment (1).

4. The osteosynthesis plate as claimed in the preceding claim, wherein the first and second segments are movably connected to each other by means of a hinge (3) .

5. The osteosynthesis plate as claimed in any of the preceding claims, wherein the first and second segments are adapted for being released and/or connected without using tools.

6. The osteosynthesis plate as claimed in any of the preceding claims, wherein the first segment (1) has a plurality of webs (21) for receiving at least one hinge wing (19).

7. The osteosynthesis plate as claimed in any of the preceding claims, wherein a plurality of segments (1, 2) can be stringed together.

8. The osteosynthesis plate as claimed in the preceding claim, wherein the second segment (2) has a web (22) for receiving a hinge wing (19) and further has a hinge opening.

9. The osteosynthesis plate as claimed in any of the preceding claims, wherein at least one passage of the first and/or the second segment has a thread, or a threaded portion, or a deformable rib.

10. A segment (2) for an osteosynthesis plate, comprising a hinge wing (19) for engaging a web (21) of another segment, and at least one passage (6) for receiving a bone screw, wherein the segment (2) is U-shaped and has a passage (6), which passage comprises a thread (11) in a lower portion thereof at a side intended to rest against a bone, wherein the two halves (15,18) of the U-shaped embodiment serve as a spring, so that a clip-like embodied hinge wing enables urging the segment (2) by widening the U-shaped embodiment and then snapping on, wherein the segment (2) has an upper half and a lower half (18, 15), and wherein the passage extents through the upper and the lover half (15,18), and wherein the passage (22) comprises a thread in the lower half at least in portions.

11. The segment (2) for an osteosynthesis plate as claimed in the preceding claim, wherein the segment comprises a web (22) for receiving a hinge wing.

12. The segment (2) for an osteosynthesis plate as claimed in any of the two preceding claims, wherein:
the segment has a concave lower surface adapted to rest against a bone; and/or
the segment has at least two spaced-apart hinge wings (19); and/or
the segment (2) is U-shaped so as to comprise an upper half and a lower half (18, 15) with a recess therebetween which enlarges to define a resilient portion on the closed side thereof; and/or
the segment (2) has a U-shaped cross section with an open side formed as a hinge wing (19); and/or
the segment is formed integrally; and/or
the segment is made of metal, in particular titanium, or titanium alloy, or stainless steel; and/or
the segment has a clip-like configuration.

## Revendications

1. Plaque d'osthéosynthèse, comprenant un premier segment (1), qui est conçu comme plaque osseuse avec une pluralié de passages (5) pour des vis à os (30), et au moins un deuxième segment (2) qui est conçu en forme de U et qui présente au moins un autre passage (6) pour une vis à os (7), **caractérisée en ce que** le deuxième segment (2) peut être relié de façon amovible au premier segment (1) et **en ce que** l'angle dans lequel sont disposés le premier et le deuxième segment parallèlement l'un à l'autre, est modifiable, le premier et le deuxième segment étant enclenchables l'un dans l'autre comme une agrafe, le deuxième segment (2) présentant une moitié supérieure (18) et une moitié inférieure (15) et pouvant être fixé solidairement sur une entretoise (21) du premier segment (1) par un rapprochement des moitiés supérieure et inférieure,
et le premier et le deuxième segment (1, 2) pouvant être fixés l'un contre l'autre par l'introduction d'une vis (7) dans le filetage d'un passage (6) du deuxième segment (2) .

2. Plaque d'osthéosynthèse selon la revendication précédente, **caractérisée en ce que** le deuxième segment (2) est conçu comme agrafe, en particulier **en ce que** le deuxième segment (2) est conçu en forme de U.

3. Plaque d'osthéosynthèse selon l'une des revendications précédentes, **caractérisée en ce que** le deuxième segment (2) est disposé de manière pivotante sur le premier segment (1).

4. Plaque d'osthéosynthèse selon la revendication précédente, **caractérisée en ce que** le premier et le deuxième segment sont reliés de manière mobile au moyen d'une charnière (3).

5. Plaque d'osthéosynthèse selon l'une des revendications précédentes, **caractérisée en ce que** le premier et le deuxième segment sont conçus pour pouvoir être séparés et/ou reliés sans outil.

6. Plaque d'osthéosynthèse selon l'une des revendications précédentes, **caractérisée en ce que** le premier segment (1) présente une pluralité d'entretoises (21) pour la prise au moins d'une aile de charnière (19).

7. Plaque d'osthéosynthèse selon l'une des revendications précédentes, **caractérisée en ce qu'**un grand nombre de segments (1, 2) sont juxtaposables.

8. Plaque d'osthéosynthèse selon la revendication précédente, **caractérisée en ce que** le deuxième segment (2) présente une entretoise (22) pour la prise d'une aile de charnière (19) ainsi qu'une ouverture à charnière.

9. Plaque d'osthéosynthèse selon l'une des revendications précédentes, **caractérisée en ce qu'**au moins un passage du premier et/ou du deuxième segment est conçu comme filetage, segment fileté ou nervure déformable et/ou **en ce que** le premier et le deuxième segment peuvent être fixés l'un contre l'autre par l'introduction d'une vis à os dans un passage du deuxième segment.

10. Segment (2) pour une plaque d'osthéosynthèse, comprenant une aile de charnière (19), destinée à être prise dans une entretoise (21) d'un autre segment, et au moins un passage (6) pour la prise d'une vis à os, le segment (2) étant conçu en forme de U et présentant un passage (6), le passage présentant, sur une face inférieure conçue pour être posée sur un os, un filetage (11), le segment (2) étant conçu en forme de U, les deux moitiés (15, 18) de la configuration en U faisant office de ressort, de telle sorte qu'une aile de charnière conçue comme agrafe permette que le segment (2) puisse être poussé par un élargissement de la configuration en U, puis s'enclenche, le segment présentant une moitié supérieure et une moitié inférieure (15, 18), et le passage (22) s'étendant par la moitié supérieure et la moitié inférieure (15, 18), et le passage (22) présentant, dans la moitié inférieure (15), au moins en partie un filetage.

11. Segment (2) pour une plaque d'osthéosynthèse selon l'une des revendications précédentes, **caractérisé en ce qu'**un segment présente une entretoise (22) pour la prise d'une aile de charnière.

12. Segment (2) pour une plaque d'osthéosynthèse selon l'une des revendications précédentes, **caractérisé en ce qu'**une face inférieure conçue pour être placée sur un os est de forme concave,
et/ou que le segment présente au moins deux ailes de charnière (19) espacées l'une de l'autre,
et/ou que le segment (2) est conçu en forme de U, l'évidement ainsi formé entre une moitié supérieure et une moitié inférieure (18, 15) présente, sur la face fermée, une section à ressort s'agrandissant,
et/ou que le segment (2) est conçu avec une paroi lisse (9) sur une face supérieure opposée,
et/ou que le segment présente une section en U, la face ouverte étant conçue comme aile de charnière (19),
et/ou que le segment est conçu en une pièce,
et/ou que le segment se compose de métal, en particulier de titane, d'un alliage de titane ou d'acier inoxydable, et/ou que le segment est conçu comme agrafe.
